# EUROPEAN PATENT APPLICATION

(11) **EP 0 611 035 A1**
(43) Date of publication of application: **17.08.1994**
(21) Application number: 94300211.3
(22) Date of filing: 12.01.1994
(51) Int. Cl.: A61M 5/315

(54) **Improvement relating to devices for generating intermittent motion**

(30) Priority: 12.01.1993 GB 9300567
(71) Applicant: OWEN MUMFORD LIMITED, Woodstock, Oxford OX20 1TU (GB)
(72) Inventor: Crossman, David Danvers, Christmas Common, Oxford, 0X9 5HL (GB); Marshall, Jeremy, Jericho, Oxford, 0X2 6DD (GB)
(74) Representative: Lainé, Simon James

(57) **Abstract**

A device for generating intermittent motion has two members (1,2; 12,13) which mutually telescope. The inner member (1,13) has an external non-helical track (4,5; 7) which steps, zig-zags or undulates and with which a follower (6) on the interior of the outer member (2; 12) co-operates. The mutual telescoping is therefore forced to take an uneven course. When incorporated in a dispensing device, such as a medical injector, the track geometry can be designed to give measured, intermittent doses from a single capsule. A quick-return track 8 can be provided to bring the members back to the initial position.

## Description

This invention concerns devices for generating intermittent motion. It may have many applications, but it originated in the context of mixing or dispensing where a capsule needed to be squeezed, but where a continuous pressure did not always suit. For example, it might allow too rapid an action, or it could be difficult to sustain. For some operations, it may be advantageous to break up squeezing into periods of pressure interposed with periods of relaxation.

According to the present invention there is provided a device for generating intermittent or non-uniform motion comprising two mutually telescoped members, the interior of the outer member and the exterior of the inner member having co-operating guide formations whereby the extent of telescoping is adjustable by mutually rotating the members about their common axis and by mutually shifting the members axially, the rotation and axial shifting either being separate operations or having a variable relationship.

For separate operations, in one preferred form the guide formations comprise annular ribs with gaps on one member, and a projection on the other member engageable between any adjacent pair of ribs and passable through said gaps. There could be a plurality of such projections, in which case they will be arranged to register with different gaps simultaneously. For ease of construction, it will generally be preferred to have the annular ribs on the inner member and the or each projection on the outer one.

Alternatively, the guide formations may comprise a track in one member with alternating portions having angled transitions between them, and a follower on the other member engaged with the track. The alternating portions may be circumferential and axially parallel, forming a stepped track. This could follow the general line of a helix, winding around said one member, or it could be of embattled form, following a generally straight path. In other arrangements, the portions may be symmetrically helical one way and then the other, or the track may be of asymmetric saw tooth form. In all these cases, the track is conveniently a channel, and the follower is a projection, or more than one projection, that engages in the channel. Again, it will usually be preferred to have the track on the inner member and the or each projection on the outer one.

The guide formations could alternatively comprise a non-helical but smoothly curved track in one member and a follower on the other member engaged with the track. Thus, instead of strictly separating axial from rotary movements, they may be carried out simultaneously, but not in the manner of a uniform screw thread.

Using convenient heraldic terms to describe the contours of possible tracks, one could have, for example, Indented, Engrailed or Invected, Dancetty, Wavy or Undy, Embattled Grady, and Urdy or Vallary.

There may be circumstances where it is advantageous to have intermittent regression in a general movement in one direction. Thus, one could have tracks of different heraldic forms, such as Dovetailed, Nebuly, Raguly, Crested, Rayonné, Potenty and Saxonised.

It will be appreciated that some of these configurations will provide a self-locking or anti-reverse feature under conditions of axial load.

In one preferred form the track is confined between radii from the axis of the device, and a further substantially straight and axially parallel track receptive to the follower is provided in said one member outside those radii and is in communication at least at one end with the first track, whereby reverse telescoping motion is substantially direct, without significant mutual twisting of said members.

Thus, at the end of the first stage of rotation and axial movement, the members may be mutually rotated through a larger angle than previous steps to bring the follower into registry with a straight, axially parallel track separate from the crooked or zig-zag one. The members can then rapidly return to their original relative axial positions. This could be particularly useful for injection devices, and would enable the parts to be more readily disassembled.

As mentioned above, this is primarily intended for dispensing devices, in which case the two members are adapted to contain a charge that is compressed axially when the two members are telescoped together by the mutual rotation and axial shifting, and which is thereby dispensed in non-uniform manner from one end of the device.

For a better understanding of the invention, some embodiments will now be described, by way of example, with reference to the accompanying drawings, in which:

Figure 1 is an exploded perspective view of a telescopic device for generating intermittent motion.

Figure 2 shows developments of outer surfaces of various inner telescopic members.

Figure 3 is a detail of one of the guide tracks shown in Figure 2, and

Figure 4 is a simplified side view of a dispensing device.

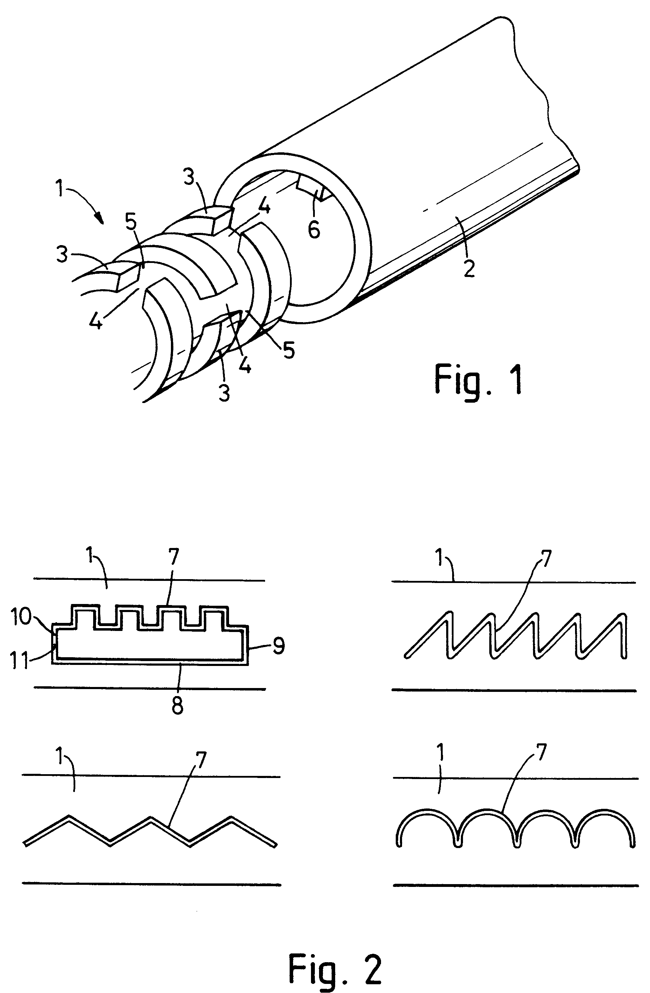

The device of Figure 1 has two cylindrical members 1 and 2 which can be mutually telescoped. The inner member 1 has a series of evenly spaced almost complete annular ribs 3, with gaps 4 providing communication between the annular grooves 5 formed by the ribs. These gaps are not aligned: each gap is circumferentially offset from the adjacent one or ones. The outer member 2 has a projection 6 extending radially inwards. This can pass through any gap 5 when the members are sleeved together, and be closely confined within any of the grooves 5. Thus to telescope the members together there must be successive and quite separate axial and rotational movements.

Referring to Figure 2, there is shown a representative sample of alternative channel tracks 7 to guide a follower such as the projection 6, but it will be understood that there can be many more. In each illustrated case, the track 7 does not twist completely around the inner member; it zig-zags in some form and is always confined within certain radii. This leaves room for a straight track 8 alongside, in communication with the track 7 at both ends via short circumferential channels 9 and 10. Thus the members can be telescoped together (or apart) in intermittent movements, while at the end of this travel, with a further mutual twist, they can be rapidly returned to their initial position with the projection 6 following the straight track 8.

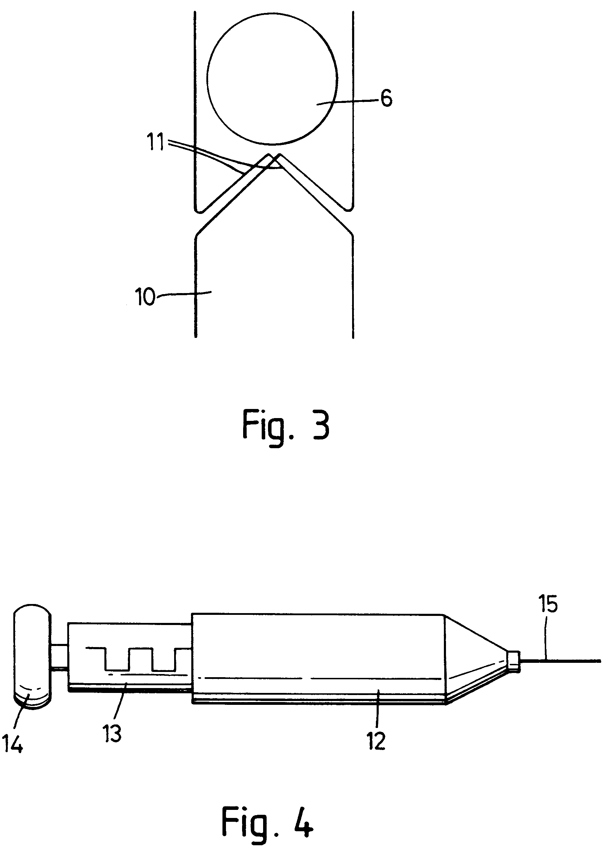

It may be desirable to confine this quick return to one direction only. Therefore, as shown in Figure 3, the channel 10 may be provided with a one-way gate 11. If the member 1 is moulded in a suitable plastics material, this gate may be formed by two integral flaps projecting from the sides of the channel 10 to meet, or almost meet, in a mitred arrangement pointing circumferentially towards the track 7. The projection 6 can flex these aside and pass from the track 8 to the track 7, but will be blocked when trying to go in the opposite direction.

There could be a similar gate in the channel 9 allowing passage of the projection only from the track 7 to the track 8.

Referring to Figure 4, a dispensing device has a barrel 12 which is equivalent to the outer member 2, and a plunger 13 which is equivalent to the member 1. This is manipulated by a knob 14 at its exposed end, and at the far end of the barrel 12 there is a needle 15. Within the barrel 12 there is a capsule (not shown) whose charge is squeezed in steps by the progressive telescoping of the plunger 13 into the barrel 12. It will be seen that, with the stepped track shown, regulated, separate small doses will be dispensed.

Instead of direct manual operation of the plunger, this assembly may be incorporated in a larger body, with spring urging of the plunger and trigger release for each dispensing operation.

## Claims

1. A device for generating intermittent or non-uniform motion comprising two mutually telescoped members, (1,2; 12,13) the interior of the outer member (2,12) and the exterior of the inner member (1,13) having co-operating guide formations whereby the extent of telescoping is adjustable by mutually rotating the members about their common axis, and by mutually shifting the members axially, the rotation and axial shifting either being separate operations or having a variable relationship.

2. A device as claimed in Claim 1, characterised in that the guide formations comprise annular ribs (3) with gaps (4) on one member (1) and a projection (6) on the other member (2) engageable between any adjacent pair of ribs (3) and passable through said gaps (4).

3. A device as claimed in Claim 2, characterised in that the annular ribs (3) are on the inner member (1) and the projection (6) is on the outer member (2).

4. A device as claimed in Claim 1, characterised in that the guide formations comprise a track (7) in one member (1) with alternating portions having angled transitions between them, and a follower on the other member engaged with the track.

5. A device as claimed in Claim 4, characterised in that the alternating portions are circumferential and axially parallel, forming a stepped track.

6. A device as claimed in Claim 1, characterised in that the guide formations comprise a non-helical but smoothly curved track in one member and a follower on the other member engaged with the track.

7. A device as claimed in Claim 5, 6 or 7, characterised in that the track (7) is confined between radii from the axis of the device and wherein a further substantially straight and axially parallel track (8) receptive to the follower is provided in said one member outside those radii and is in communication at least at one end with the first track (7), whereby reverse telescoping motion is substantially direct, without significant mutual twisting of said member.

8. A device as claimed in Claim 8, characterised in that the further track (8) is in communication with the first track (7) at both ends.

9. A device as claimed in Claim 7 or 8, characterised in that non-return means (11) allow the follower (6) to pass from the straight track (8) to the first track (7) at the other end but not to reverse that movement.

10. A dispensing device comprising a device as claimed in any preceding claim in which the two members (12,13) are adapted to contain a charge that is compressed axially when the two members (12,13) are telescoped together by the mutual rotation and axial shifting, and which is thereby dispensed in non-uniform manner from one end (15) of the device.
